# EUROPÄISCHE PATENTANMELDUNG

(11) **EP 0 794 165 A1**
(43) Veröffentlichungstag der Anmeldung: **10.09.1997**
(21) Anmeldenummer: 97103446.7
(22) Anmeldetag: 03.03.1997
(51) Int. Cl.: C07B 35/02, C07C 13/465, C07C 1/22

(54) **Verfahren zur Herstellung von Indenen**

(30) Priorität: 07.03.1996 DE 19608814
(71) Anmelder: HOECHST AKTIENGESELLSCHAFT, 65929 Frankfurt am Main (DE)
(72) Erfinder: Weiguny, Jens, Dr., 67251 Freinsheim (DE); Borchert, Holger, Dr., 65929 Frankfurt (DE); Dingerdissen, Uwe, Dr., 64342 Seeheim-Jugenheim (DE)

(57) **Zusammenfassung**

Die vorliegende Erfindung betrifft somit ein Verfahren zur Herstellung eines Indens, wobei ein Indanon mit Wasserstoff umgesetzt wird, in Gegenwart eines Katalysators enthaltend eines oder mehrere Oxide, bevorzugt Metalloxide.

Bevorzugte Oxide enthalten Elemente der Gruppen Ia, IIa, IIIa, IVa, Va, VIa, Ib, IIb, IIIb, IVb, Vb, VIb, VIIb und VIII des Periodensystems, oder der Elemente der Gruppe der Lanthaniden.

## Beschreibung

Die vorliegende Erfindung betrifft ein technisch einfaches Verfahren zur Herstellung von Indenen in Gegenwart von Oxidkatalysatoren, insbesondere Metalloxidkatalysatoren.

Indene sind wichtige Zwischenprodukte bei der Herstellung von Metallocen-Komplexen, wobei Indene als Ligandensystem zum Aufbau von Metallocen-Komplexen verwendet werden (EP-A 336 128). Insbesondere verbrückte, chirale Bis-indenyl-Zirkonocene besitzen als hochaktive Katalysatoren bei der Olefinpolymerisation große Bedeutung (vgl. EP-A 129 368; EP-A 321 852). Durch Variation des Ligandensystems, z.B. durch Substitution, können die Katalysatoreigenschaften gezielt beeinflußt werden. Hierdurch ist es möglich, die Polymerausbeuten, die Molmasse, die Taktizität oder den Schmelzpunkt der Polymere in gewünschten Maß zu verändern (New J. Chem. 1990, 14, 499; Organomet. 1990, 9, 3098; Angew. Chem. 1990, 102, 339; EP-A 316 155; EP-A 351 392).

In der Literatur sind Verfahren zur Herstellung von substituierten Indenen aus den entsprechenden Indanonen beschrieben. Bei einem dieser Verfahren wird zunächst ein Indanon mit einem Reduktionsmittel wie NaBH₄ (J. Med. Chem. 1990, 33, 758), LiAlH₄ (Bull. Soc. Chim. Fr. 1973, 11, 3092) oder KBH₄ (J. Med. Chem. 1991, 34, 919) zum entsprechenden Alkohol reduziert. Diese Reduktion gelingt auch, wenn sie nach Meerwein-Ponndorf-Verley mit Al(i-Pr)₃ (Bull. Soc. Chim. Fr. 1971, 1351) durchgeführt wird. In manchen Fällen gelingt auch eine Hydrierung mit Wasserstoff an einem Raney-Nickel-Katalysator. Allerdings läßt sich zum Beispiel 2-Methylinden gar nicht umsetzen (Houben Weyl 4/1c S. 205). Der Alkohol wird dann in einem zweiten Schritt durch Eliminierung von Wasser in das Inden überführt. Hierzu benötigt man saure Katalysatoren wie Essigsäure (J. Org. Chem. 1992, 57, 2), p-Toluolsulfonsäure (Organomet. 1990, 9, 3098), HCl (Bull. Soc. Chim. Fr. 1971, 1351), H₂SO₄ (Synlett 1991, 9, 642) oder KHSO₄ (Bull. Soc. Chim. Fr. 1973, 11, 3092). Alternativ zu sauren Katalysatoren können auch wasserentziehende Mittel wie P₂O₅ eingesetzt werden (J. Med. Chem. 1991, 34, 919).

In einem anderen Verfahren wird das Indanon in einer Wolff-Kizner-Reduktion mit Hydrazin in Gegenwart von Natriumalkoholat zum entsprechenden Indan umgesetzt. Dieses wird dann in einem zweiten Schritt mit 2,3-Dichlor-5,6-dicyano-p-benzo- chinon (DDQ) zum Inden dehydriert (J. Org. Chem. 1990, 55, 756).

Die Verfahren des Standes der Technik haben den Nachteil, daß sie zweistufig und damit sehr aufwendig sind. Außerdem werden im erstgenannten Verfahren teure Reagenzien wie NaBH₄ oder LiAlH₄ im Überschuß verwendet, was wiederum zu einem erheblichen Abfallaufkommen führt. Die anschließende Dehydratisierung hat das Problem, daß es durch erhöhte Temperatur und den sauren Katalysator zu Polymerisation kommen kann, oder daß wasserentziehende Mittel eingesetzt werden müssen. Im zweitgenannten Verfahren werden zur Reduktion das giftige Hydrazin und zur Dehydrierung äquimolare Mengen von giftigem und teurem DDQ verwendet.

Es bestand somit die Aufgabe, ein Verfahren zur Herstellung von Indenen zu finden, das die aus dem Stand der Technik bekannten Nachteile vermeidet.

Es wurde nun überraschend gefunden, daß Indanone mit Wasserstoff an einem Oxidkatalysator in einem Reaktionsschritt zu Indenen überführt werden können.

Die vorliegende Erfindung betrifft somit ein Verfahren zur Herstellung eines Indens, wobei ein Indanon mit Wasserstoff umgesetzt wird, in Gegenwart eines Katalysators enthaltend eines oder mehrere Oxide, insbesondere Metalloxide.

Bevorzugt sind Oxide von Elementen der Gruppen Ia, IIa, IIIa, IVa, Va, VIa, Ib, IIb, IIIb, IVb, Vb, VIb, VIIb und VIII des Periodensystems der Elemente, oder Oxide der Elemente der Gruppe der Lanthaniden. Bevorzugte Katalysatoren sind Oxide, insbesondere Metalloxide der Formel (I),

ZnₐM¹_{b}M²_{c}Oₓ (I)

worin M¹ für mindestens ein Element aus der Gruppe bestehend aus Aluminium, Zirkon, Titan und Silizium und M² für mindestens ein Element aus der Gruppe bestehend aus Cadmium, Kupfer, Silber, Nickel, Cobalt, Eisen, Mangan, Chrom, Molybdän, Tantal, Scandium, Wolfram, Vanadium, Niob, Hafnium, Yttrium, Bor, Indium, Zinn, Blei, Wismut, Selen, Gallium, Germanium, Antimon, Arsen, Tellur oder ein Element der Lanthanide steht,
a = 1 ist, b einen Wert im Bereich von 0 bis 20, bevorzugt 0,01 bis 20, besonders bevorzugt von 0,1 bis 10, und c einen Wert im Bereich von 0 bis 3, bevorzugt von 0,01 bis 2, hat, und x einen Wert hat, der sich entsprechend der Stöchiometrie und den Oxidationszuständen der Metalle Zn, M¹ und M² ergibt.

Die Buchstaben a, b, c und x geben das Atomverhältnis der jeweiligen Elemente an. a, b, c und x können gleiche und unterschiedliche Werte annehmen. Beispiele für Oxide der Formel (I) sind ZnO, ZnₐAl_{b}Oₓ, ZnₐAl_{b}Co_{c}Oₓ, ZnₐAl_{b}Ni_{c}Oₓ, ZnₐAl_{b}Ce_{c}Oₓ, Znₐ(AlSi)_{b}Oₓ, Znₐ(AlTi)_{b}Oₓ, Znₐ(AlZr)_{b}Oₓ, wobei bevorzugt a = 1, 1 ≤ b ≤ 3, 0,01 ≤ c ≤ 0,1 und x sich entsprechend der Stöchiometrie und den Oxidationszuständen der Metalle ergibt.

In einer weiteren bevorzugten Ausführungsvariante der vorliegenden Erfindung können anstatt Oxide der Formel (I) Oxide der Formel (II) eingesetzt werden,

M³₂M⁴O₄ (II)

bei denen es sich um Doppeloxide aus der Verbindungsklasse der Spinelle handelt (Chemie der Elemente, N.N. Greenwood, A. Earnshaw, VCH-Verlagsgesellschaft mbH, Weinheim, 1990; Lehrbuch der Anorganischen Chemie, A.F. Holleman, N. Wiberg, de Gruyter, Berlin, 1985). In Formel (II) können M³ und M⁴ jedes beliebige Element des Periodensystems der Elemente darstellen, wobei M³ ein Element mit der Oxidationsstufe I, II oder III darstellt und M⁴ ein Element mit der Oxidationsstufe II, III oder VI ist. Es ist bevorzugt daß, wenn M³ ein Element mit der Oxidationsstufe I darstellt, M⁴ die Oxidationsstufe VI besitzt, wenn M³ ein Element mit der Oxidationsstufe II darstellt, M⁴ die Oxidationsstufe IV besitzt, und wenn M³ ein Element mit der Oxidationsstufe III darstellt, M⁴ die Oxidationsstufe II besitzt. Es ist auch möglich, daß es sich bei M³ und M⁴ um das gleiche Element handelt, sofern es in den entsprechenden, unterschiedlichen Oxidationsstufen vorliegen kann. Beispiele für Oxide der Formel (II) sind Al₂ZnO₄, Al₂CoO₄, Al₂MnO₄, Al₂FeO₄, Al₂NiO₄ und Cr₂FeO₄.

Der erfindungsgemäß eingesetzte Katalysator kann durch Imprägnierung auf Träger und Formkörper oder durch Cofällung sowie anschließende Trocknung und Kalzination hergestellt werden. Eine weitere Möglichkeit besteht in der direkten Kalzination geeigneter Metallverbindungen, z.B. von Nitraten, Acetaten, Carbonaten oder anderen Salzen und Komplexen der Elemente M¹ bis M⁴.

Zur Imprägnierung auf Träger kann eine Lösung von Verbindungen der Elemente M¹bis M⁴ auf einen Träger, der bevorzugt anorganisch ist und beispielsweise aus SiO₂, SiC, Al₂O₃, Al(OH)₃, ZrO₂, Alumosilikaten, SiN oder TiO₂ bestehen kann, aufgebracht werden. Zur Imprägnierung geeignete Verbindungen der Elemente M¹ bis M⁴ sind z.B. deren Halogenide, Nitrate, Sulfate, Oxalate, Carboxylate und Alkoxide. Die imprägnierten Träger werden anschließend bei 100°C bis 150°C, bevorzugt bei 130°C, getrocknet und bei 400-1000°C, bevorzugt bei 500 bis 800°C kalziniert. Die so hergestellten Katalysatoren können nach üblichen Verfahren vor oder nach dem Kalzinieren zu Pellets, Tabletten oder Extrudaten weiterverarbeitet werden.

Zur Cofällung können geeignete Verbindungen der Elemente M¹ bis M⁴ bei geeigneten pH-Werten gefällt werden. Nach der Fällung werden entstandene Hydroxide abfiltriert und mit einem geeigneten Lösungsmittel gewaschen. Die Trocknung erfolgt bei 100 bis 150°C, bevorzugt bei 130°C, gegebenenfalls unter Anlegung von Vakuum, die Kalzination bei 400 bis 1000°C, bevorzugt bei 500 bis 900°C. Der so hergestellte Katalysator liegt als Granulat vor und kann nach Zerkleinerung auf die gewünschte Teilchengröße direkt in die Reaktion eingesetzt werden.

Vor dem Einsetzen in dem erfindungsgemäßen Verfahren kann der Katalysator bei Temperaturen von 100 bis 800°C mit einem geeignetem Reduktionsmittel präformiert werden. Das erfindungsgemäße Verfahren kann in kontinuierlicher oder diskontinuierlicher Fahrweise in einem geeigneten Reaktor durchgeführt werden. Es hat sich als günstig erwiesen bei einer Temperatur von 200 bis 600°C, insbesondere bei 250 bis 400°C, und einem Druck von 0.1 bis 10 bar, insbesondere bei atmosphärischem Druck, zu arbeiten.

Das erfindungsgemäße Verfahren kann mit molekularem Wasserstoff durchgeführt werden, der auch in situ hergestellt werden kann. Es ist auch möglich den Wasserstoff mit einem Inertgas wie Stickstoff oder Argon zu verdünnen. Das erfindungsgemäß eingesetzte Indanon kann als Feststoff, als Schmelze, als Flüssigkeit oder als Lösung in einem geeignetem Lösungsmittel, wie Benzol, Xylol, Toluol oder Cyclohexan eingesetzt werden. Bevorzugt wird das Indanon einem Verdampfer zugeführt und anschließend in der Gasphase mit dem erfindungsgemäß eingesetzten Katalysator in Kontakt gebracht. Das molare Verhältnis des Indanons zu Wasserstoff liegt bevorzugt bei 1:1 bis 1:500, besonders bevorzugt bei 1:2 bis 1:50. Die Zufuhrgeschwindigkeit für die Indanone liegt bevorzugt bei 0.01 bis 20 g/ml_{Katalysator}h (LHSV: liquid hourly space velocity), besonders bevorzugt bei 0.2 bis 5 g/ml_{Katalysator}h. Die Zufuhrgeschwindigkeit des Wasserstoffs liegt bevorzugt bei 50 bis 50000 h⁻¹ (GHSV: gasous hourly space velocity), besonders bevorzugt bei 100 bis 10000 h⁻¹.

In dem erfindungsgemäßen Verfahren wird bevorzugt ein Indanon der Formel (III) eingesetzt 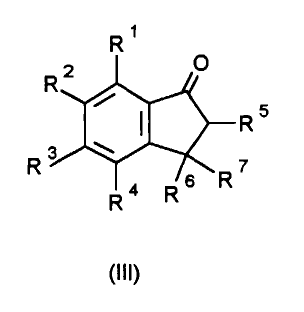 worin R¹, R², R³, R⁴, R⁵, R⁶ und R⁷ gleich oder verschieden sind und ein Wasserstoffatom, ein Halogenatom oder einen C₁-C₃₀-kohlenstoffhaltigen Rest bedeuten, wie (C₁-C₂₀)-Alkyl, (C₆-C₁₄)-Aryl, (C₁-C₁₀)-Alkoxy, (C₂-C₁₀)-Alkenyl, (C₇-C₂₀)-Arylalkyl, (C₇-C₂₀)-Alkylaryl, (C₆-C₁₀)-Aryloxy, (C₁-C₁₀)-Fluoralkyl, (C₆-C₁₀)-Halogenaryl, (C₂-C₁₀)-Alkinyl oder -SiR⁸₃, wobei R⁸ für (C₁-C₁₀)-Alkyl steht, oder zwei oder mehr Reste R¹ bis R⁷ können mit den sie verbindenden Atomen einen oder mehrere substituierte oder unsubstituierte Ringe bilden. Der C₁-C₃₀-kohlenstoffhaltige Rest kann ein heteroaromatischer Rest - bevorzugt mit 5 oder 6 Ringgliedern - sein, der eines oder mehrere Heteroatome wie Stickstoff, Phosphor oder Schwefel enthalten kann.

Alkyl steht für geradkettiges, cyclisches oder verzweigtes Alkyl. Halogen bedeutet Fluor, Chlor, Brom oder lod, insbesondere Fluor oder Chlor. Beispiele für heteroaromatische Reste sind Thienyl, Furyl oder Pyridyl.

Die von benachbarten Resten R¹ bis R⁷ gebildeten Ringe können durch Substituenten in der Bedeutung von R¹ bis R⁷, einschließlich der dafür genannten Vorzugsbereiche, substituiert sein.

In der Formel (III) gilt bevorzugt, daß R¹, R², R³ und R⁴ gleich oder verschieden sind und Wasserstoff, (C₆-C₁₀)-Aryl oder (C₁-C₁₀)-Alkyl bedeuten, oder die Reste R¹ und R², R² und R³ oder R³ und R⁴ mit den sie verbindenden Atomen einen substituierten oder unsubstituierten sechsgliedrigen, gesättigten oder ungesättigten Carbocyclus bilden, R⁵ für Methyl steht, und R⁶ und R⁷ Wasserstoff bedeuten.

Der von benachbarten Substituenten R¹-R⁴ gebildete gesättigte oder ungesättigte Fünf- oder Sechsring (Carbocyclus) kann zusätzlich Substituenten, bevorzugt (C₁-C₁₀)-Alkyl, tragen.

Bevorzugt wird mit dem erfindungsgemäßen Verfahren ein Inden der Formel (IV) oder dessen Isomere (Va) und (Vb) hergestellt, 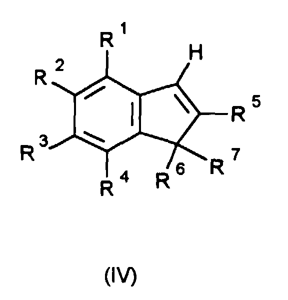 worin R¹, R², R³, R⁴, R⁵, R⁶ und R⁷ gleich oder verschieden sind und ein Wasserstoffatom, ein Halogenatom oder einen C₁-C₃₀-kohlenstoffhaltigen Rest bedeuten, wie (C₁-C₂₀)-Alkyl, (C₆-C₁₄)-Aryl, (C₁-C₁₀)-Alkoxy, (C₂-C₁₀)-Alkenyl, (C₇-C₂₀)-Arylalkyl, (C₇-C₂₀)-Alkylaryl, (C₆-C₁₀)-Aryloxy, (C₁-C₁₀)-Fluoralkyl, (C₆-C₁₀)-Halogenaryl, (C₂-C₁₀)-Alkinyl oder -SiR⁸₃, wobei R⁸ für (C₁-C₁₀)-Alkyl steht, oder zwei oder mehr Reste R¹ bis R⁷ können mit den sie verbindenden Atomen einen oder mehrere substituierte oder unsubstituierte Ringe bilden. Der C₁-C₃₀-kohlenstoffhaltige Rest kann ein heteroaromatischer Rest - bevorzugt mit 5 oder 6 Ringgliedern - sein, der eines oder mehrere Heteroatome wie Stickstoff, Phosphor oder Schwefel enthalten kann.

Alkyl steht für geradkettiges, cyclisches oder verzweigtes Alkyl. Halogen bedeutet Fluor, Chlor, Brom oder Iod, insbesondere Fluor oder Chlor. Beispiele für heteroaromatische Reste sind Thienyl, Furyl oder Pyridyl.

Die von benachbarten Resten R¹ bis R⁷ gebildeten Ringe können durch Substituenten in der Bedeutung von R¹ bis R⁷, einschließlich der dafür genannten Vorzugsbereiche, substituiert sein.

In der Formel (IV) gilt bevorzugt, daß R¹, R², R³ und R⁴ gleich oder verschieden sind und Wasserstoff, (C₆-C₁₀)-Aryl oder (C₁-C₁₀)-Alkyl bedeuten, oder die Reste R¹ und R², R² und R³ oder R³ und R⁴ mit den sie verbindenden Atomen einen substituierten oder unsubstituierten sechsgliedrigen, gesättigten oder ungesättigten Carbocyclus bilden, R⁵ für Methyl steht, und R⁶ und R⁷ Wasserstoff bedeuten.

Der von benachbarten Substituenten R¹-R⁴ gebildete gesättigte oder ungesättigte Fünf- oder Sechsring (Carbocyclus) kann zusätzlich Substituenten, bevorzugt (C₁-C₁₀)-Alkyl, tragen.

Beispiele für Indene der Formel (IV) sind Inden, 2-Methylinden, 3-Methylinden, 3-tert-butylinden, 3-Trimethylsilylinden, 2-Methyl-4-phenylinden, 2-Methyl-4-(2'-pyridyl)inden, 2-Ethyl-4-phenylinden, 2-Methyl-naphthylinden, 2-Methyl-4-isopropylinden, 2-Methyl-4,6-diisopropylinden, Benzoinden, 2-Methyl-4,5-benzoinden, 2-Methyl-acenaphthinden, 2-Ethyl-naphthylinden, 2-Ethyl-4-isopropylinden.

Zu dem Inden der Formel (IV) existiert ein Doppelbindungsisomer welches seinerseits zwei Konstitutionsisomere (Formel Va und Vb) hat.

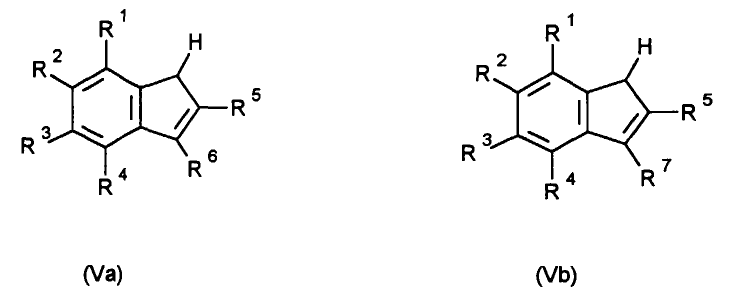

Die mit dem erfindungsgemäßen Verfahren hergestellten Indene können von Nebenprodukten durch Destillation, Säulenchromatogrophie oder Kristallisation gereinigt werden. Die erhaltenen Isomere können als Gemisch direkt für die Synthese der entsprechenden Metallocen-Komplexe eingesetzt werden. Die Synthese solcher Metallocene, ausgehend von Indenen, ist z.B. beschrieben in AU-A-31479/89; J. Organomet. Chem. 1988, 342, 21 und EP-A 284 707.

Das erfindungsgemäße Verfahren zeichnet sich dadurch aus, daß es technisch einfach durchzuführen ist und die gewünschten Indene mit sehr guten Selektivitäten liefert, bevorzugt mit Selektivitäten von 89 bis 99,9 %, besonders bevorzugt mit Selektivitäten von 95 bis 99 %.

Die nachfolgenden Beispiele dienen zur Illustration der vorliegenden Erfindung, haben jedoch keinerlei limitierenden Charakter:

### Beispiel A:

Man löst 139,0 g Al(NO₃)₃ x 9 H₂O und 55,0 g Zn(NO₃)₂ x 6 H₂O in 3,8 l Wasser, kühlt auf 5°C und stellt dem pH-Wert mit 25%iger Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Waserstoffstrom präformiert.

### Beispiel B:

Man löst 69,5 g Al(NO₃)₃ x 9 H₂O und 110,0 g Zn(NO₃)₂ x 6 H₂O in 3,8 l Wasser, kühlt auf 5°C und stellt dem pH-Wert mit 25%iger Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Waserstoffstrom präformiert.

### Beispiel C:

Man löst 139,0 g Al(NO₃)₃ x 9 H₂O und 110,0 g Zn(NO₃)₂ x 6 H₂O in 3,8 l Wasser, kühlt auf 5°C und stellt dem pH-Wert mit 25%iger Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Waserstoffstrom präformiert.

### Beispiel D:

Man löst 139,0 g Al(NO₃)₃ x 9 H₂O und 54,9 g Zn(NO₃)₂ x 6 H₂O und 2,2 g Co(NO₃)₃ x 9 H₂O in 3,8 l Wasser, kühlt auf 5°C und stellt dem pH-Wert mit 25%iger Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Waserstoffstrom präformiert.

### Beispiel E:

Man löst 139,0 g Al(NO₃)₃ x 9 H₂O und 54,9 g Zn(NO₃)₂ x 6 H₂O und 1,35 g Cu(NO₃)₂ x 3 H₂O in 3,8 l Wasser, kühlt auf 5°C und stellt dem pH-Wert mit 25%iger Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Waserstoffstrom präformiert.

### Beispiel F:

Man löst 139,0 g Al(NO₃)₃ x 9 H₂O und 54,9 g Zn(NO₃)₂ x 6 H₂O und 2,23 g Cr(NO₃)₃ x 9 H₂O in 3,8 l Wasser, kühlt auf 5°C und stellt dem pH-Wert mit 25%iger Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Waserstoffstrom präformiert.

### Beispiel G:

Man löst 139,0 g Al(NO₃)₃ x 9 H₂O und 54,9 g Zn(NO₃)₂ x 6 H₂O und 1,62 g Ni(NO₃)₂ x 6 H₂O in 3,8 l Wasser, kühlt auf 5°C und stellt dem pH-Wert mit 25%iger Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Waserstoffstrom präformiert.

### Beispiel H:

Man löst 139,0 g Al(NO₃)₃ x 9 H₂O und 54,9 g Zn(NO₃)₂ x 6 H₂O und 1,40 g Mn(NO₃)₂ x 4 H₂O in 3,8 l Wasser, kühlt auf 5°C und stellt dem pH-Wert mit 25%iger Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Waserstoffstrom präformiert.

### Beispiel I:

Man löst 139,0 g Al(NO₃)₃ x 9 H₂O und 54,9 g Zn(NO₃)₂ x 6 H₂O und 2,03 g Y(NO₃)₃ x 6 H₂O in 3,8 l Wasser, kühlt auf 5°C und stellt dem pH-Wert mit 25%iger Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Waserstoffstrom präformiert.

### Beispiel J:

Man löst 139,0 g Al(NO₃)₃ x 9 H₂O und 54,9 g Zn(NO₃)₂ x 6 H₂O und 2,25 g Fe(NO₃)₃ x 9 H₂O in 3,8 l Wasser, kühlt auf 5°C und stellt dem pH-Wert mit 25%iger Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Waserstoffstrom präformiert.

### Beispiel K:

Man löst 139,0 g Al(NO₃)₃ x 9 H₂O und 54,9 g Zn(NO₃)₂ x 6 H₂O und 2,42 g Ce(NO₃)₃ x 6 H₂O in 3,8 l Wasser, kühlt auf 5°C und stellt dem pH-Wert mit 25%iger Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Waserstoffstrom präformiert.

### Beispiel L:

Man löst 139,0 g Al(NO₃)₃ x 9 H₂O und 54,9 g Zn(NO₃)₂ x 6 H₂O und 1,16 g Tetraethoxysilan in 3,8 l Wasser, kühlt auf 5°C und stellt dem pH-Wert mit 25%iger Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Waserstoffstrom präformiert.

### Beispiele 1-13:

In einem Rohrreaktor wurde 2-Methylindanon in Gegenwart der Katalysatoren aus den Beispielen A-L (Beispiele 1-12) bzw. in Gegenwart eines ZnO-Katalysators (Beispiel 13) mit Wasserstoff zu 2-Methylinden umgesetzt. Das Schüttvolumen des eingesetzten Katalysators betrug 20 ml. Die Verdampfung des eingesetzten 2-Methylindanons erfolgte in einem vorgeschaltetem Verdampfer. GHSV (gaseous hourly space velocity), LHSV (liquid hourly space velocity) und Reaktionstemperatur sind in Tabelle 1 angegeben. Das Reaktionsprodukt wurde mit einem Kühler kondensiert und gaschromatographisch analysiert.

**Tabelle 1**

| Bsp. | Katalysator aus Beispiel | GHSV [h⁻¹] | LHSV [g/ml_{Kat.}h] | Temperatur | Umsatz | Selektivität |
|---|---|---|---|---|---|---|
| 1 | A | 1250,00 | 0,61 | 350°C | 100% | 98% |
| 2 | B | 1250,00 | 0,20 | 350°C | 100% | 95% |
| 3 | C | 1250,00 | 0,38 | 350°C | 100% | 95% |
| 4 | D | 1250,00 | 0,38 | 310°C | 100% | 99% |
| 5 | E | 1250,00 | 0,38 | 330°C | 97% | 92% |
| 6 | F | 1250,00 | 0,38 | 330°C | 98% | 92% |
| 7 | G | 1250,00 | 0,38 | 310°C | 100% | 99% |
| 8 | H | 1250,00 | 0,38 | 350°C | 100% | 92% |
| 9 | I | 1250,00 | 0,38 | 350°C | 100% | 89% |
| 10 | J | 1250,00 | 0,38 | 310°C | 97% | 93% |
| 11 | K | 1250,00 | 0,38 | 310°C | 100% | 99% |
| 12 | L | 1250,00 | 0,38 | 310°C | 99% | 94% |
| 13 | ZnO-Katalysator | 1250,00 | 0,38 | 330°C | 100% | 99% |

## Patentansprüche

1. Verfahren zur Herstellug eines Indens, wobei ein Indanon mit Wasserstoff umgesetzt wird, in Gegenwart eines Katalysators enthaltend eines oder mehrere Oxide, bevorzugt Metalloxide.

2. Verfahren gemäß Anspruch 1, worin das Oxid ein Element der Gruppen Ia, IIa, IIIa, IVa, Va, VIa, Ib, IIb, IIIb, IVb, Vb, VIb, VIIb und VIII des Periodensystems der Elemente, oder ein Element der Gruppe der Lanthaniden enthält.

3. Verfahren gemäß Anspruch 1 oder 2, worin das Oxid eine Verbindung der Formel (I) ist,
ZnₐM¹_{b}M²_{c}Oₓ (I)
worin M¹ für mindestens ein Element aus der Gruppe bestehend aus Aluminium, Zirkon, Titan und Silizium und M² für mindestens ein Element aus der Gruppe bestehend aus Cadmium, Kupfer, Silber, Nickel, Cobalt, Eisen, Mangan, Chrom, Molybdän, Tantal, Scandium, Wolfram, Vanadium, Niob, Hafnium, Yttrium, Bor, Indium, Zinn, Blei, Wismut, Selen, Gallium, Germanium, Antimon, Arsen, Selen, Tellur oder ein Element der Lanthanide steht,
a = 1 ist, b einen Wert im Bereich von 0,01 bis 20, bevorzugt von 0,1 bis 10, und c einen Wert im Bereich von 0 bis 3, bevorzugt von 0,01 bis 2, hat, und x einen Wert hat der sich entsprechend der Stöchiometrie und den Oxidationszuständen der Metalle Zn, M¹ und M² ergibt.

4. Verfahren gemäß Anspruch 1 oder 2, worin das Oxid eine Verbindung der Formel (II) ist,
M³₂M⁴O₄ (II)
worin M³ und M⁴ jedes beliebige Element des Periodensystems der Elemente darstellen können, und M³ ein Element mit der Oxidationsstufe I, II oder III ist und M⁴ ein Element mit der Oxidationsstufe II, III oder VI ist.

5. Verfahren gemäß Anspruch 4, worin in Formel II, wenn M³ ein Element mit der Oxidationsstufe I darstellt, M⁴ die Oxidationsstufe VI besitzt, wenn M³ ein Element mit der Oxidationsstufe II darstellt, M⁴ die Oxidationsstufe IV besitzt, und wenn M³ ein Element mit der Oxidationsstufe III darstellt, M⁴ die Oxidationsstufe II besitzt.

6. Verfahren gemäß einem oder mehreren der Ansprüche 1 bis 5, worin ein Indanon der Formel (III) eingesetzt wird 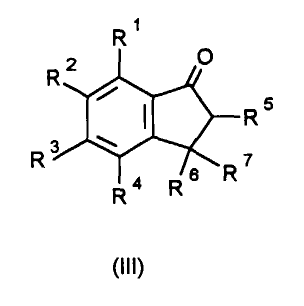 worin R¹, R², R³, R⁴, R⁵, R⁶ und R⁷ gleich oder verschieden sind und ein Wasserstoffatom, ein Halogenatom oder einen C₁-C₃₀-kohlenstoffhaltigen Rest bedeuten, oder zwei oder mehr Reste R¹ bis R⁷ können mit den sie verbindenden Atomen einen oder mehrere substituierte oder unsubstituierte Ringe bilden.

7. Verwendung eines Katalysators, enthaltend eines oder mehrere Oxide, bevorzugt Metalloxide, zur Herstellung eines Indens.
